# EUROPEAN PATENT APPLICATION

(11) **EP 2 529 750 A1**
(43) Date of publication of application: **05.12.2012**
(21) Application number: 11173119.6
(22) Date of filing: 23.10.2009
(51) Int. Cl.: A61K 39/05, A61K 39/08, A61K 39/10, A61K 39/102, A61K 39/13, A61K 39/29

(54) **Combination vaccine with whole cell pertussis**

(30) Priority: 24.10.2008 IN DE24372008
(62) Divisional of application: 09821705.2
(71) Applicant: Panacea Biotec Ltd., New Delhi 110 044 (IN)
(72) Inventor: Jain, Rajesh, 110 044 NEW DELHI (IN); Singh, Sukhjeet, 110 044 NEW DELHI (IN); Jambu, Lavit, 110 044 NEW DELHI (IN)
(74) Representative: Lecca, Patricia S.

(57) **Abstract**

The present invention relates to a combination vaccine comprising a mixture of antigens for protection against diseases such as diphtheria, tetanus, whole cell pertussis and polio. The present invention also relates to inclusion of one or more antigens in the said combination vaccine, for protection against infections caused by *Haemophilus influenzae,* Hepatitis virus, and other pathogens, such that administration of the vaccine can simultaneously immunize a subject against more than one pathogen. The invention in particular relates to a fully liquid stable combination vaccine comprising the antigens as indicated above and the methods for manufacturing the same.

## Description

### FIELD OF INVENTION

The present invention relates to a combination vaccine comprising a mixture of antigens for protection against diseases such as diphtheria, tetanus, pertussis and polio. The present invention also relates to inclusion of one or more antigens in the said combination vaccine, for protection against infections caused by *Haemophilus influenzae,* Hepatitis virus, and other pathogens, such that administration of the vaccine can simultaneously immunize a subject against more than one pathogen. The invention in particular relates to a fully liquid stable combination vaccine comprising the antigens as indicated above and the methods for manufacturing the same.

### BACKGROUND OF THE INVENTION

### Antigens of the vaccine

### Diphtheria and Tetanus antigens

Diphtheria and tetanus are acute infections caused by *Cornyebacterium diphtheriae* and *Clostridium tetani,* respectively. The toxins of these bacteria are the major cause of the respective diseases. The vaccines affording protection against these bacteria contain these toxins that are toxoided to lose their infectivity. The toxins are treated using chemicals such as formaldehyde or glutaraldehyde for making toxoids [diphtheria toxoid (DT) and Tetanus toxoid (TT)]. CRM 197, a mutant diphtheria toxin, is also used in certain vaccines.

### Pertussis antigens

The whooping cough disease or the pertussis is caused by *Bordetella pertussis.* This is a debilitating and serious disease that may even lead to death. The initial vaccines against the disease were based on the whole cells which were treated with chemicals such as formaldehyde to kill the cells and inactivate the toxic materials. Efforts to develop an inactivated whole-cell pertussis vaccine began soon after *B. pertussis* was grown in pure culture in 1906. In the 1920s Dr. Louis W. Sauer developed a vaccine for whooping cough. In 1925, the Danish physician Thorvald Madsen was the first to test a whole-cell pertussis vaccine on a wide scale. In 1942, the American scientist Pearl Kendrick combined the whole-cell pertussis vaccine with diphtheria and tetanus toxoids to generate the first DTP combination vaccine. These vaccines called as the 'whole cell (wP) vaccines' were highly efficacious.

The later vaccine called the 'component vaccine' comprised lesser number of highly purified antigens. Many virulence associated factors have been suggested for the inclusion in the 'acellular vaccine', which is less defined as compared to component vaccine. The vaccines comprising only PT were partially protective, whereas the vaccines comprising the combination of PT/FHA were more efficacious but still had lesser immunogenicity than the wP vaccines.

### Poliomyelitis antigens

Two different kinds of vaccine are available:
- A live attenuated (weakened) oral polio vaccine (OPV) developed by Dr. Albert Sabin in 1961. OPV, comprising the Sabin strains, is given orally.
- An inactivated (killed) polio vaccine (IPV) developed in 1955 by Dr. Jonas Salk. IPV, comprising the Salk strains, is given as an injection.

Both live attenuated (OPV) and inactivated (IPV) polio vaccines have been effective in controlling the polio disease worldwide. The polio vaccine may comprise the Salk or the Sabin strains. Mahoney type 1, MEF Type 2 and the Saukett type 3 are the Salk strains that have been used in the vaccine against the poliomyelitis disease. The Sabin strains include the Sabin 1 and Sabin 2 strains.

### Haemophilus influenzae (Hib) antigens

*Haemophilus influenzae* is a Gram-negative coccobacillus that is a normal part of upper respiratory tract flora. *Haemophilus influenzae* type b (Hib b) is a major cause of meningitis invasive bloodborne infections in young children and major cause of meningitis in the first 2 years of life. Immunization against *Haemophilus influenzae* began in Canada in 1987 with a polysaccharide vaccine [polyribose ribitol phosphate (PRP)]. The polyribosylribitol phosphate (PRP) capsule of Hib is a major virulence factor for the organism. Antibody to PRP is the primary contributor to serum bactericidal activity, and increasing levels of antibody are associated with decreasing risk of invasive disease. PRP is a T-cell independent antigen and hence is characterized by a) induction of a poor antibody response in less than 18-month-old infants and children, b) a variable and quantitatively smaller antibody response than that seen with T-cell dependent antigens, c) production of a higher proportion of immunoglobulin M (IgM), and d) inability to induce a booster response.

The initial vaccines based only on the PRP component proved to be ineffective in the infants. Further efforts were directed towards the PRP conjugate vaccine, wherein the PRP is conjugated to proteins called the carrier proteins such as the outer membrane protein of *Neisseria meningitides,* diphtheria toxoid, tetanus toxoid and CRM 197.

### Hepatitis (Hep) antigens

There are various strains of Hepatitis virus. Hepatitis B is a disease caused by hepatitis B virus (HBV) which infects the liver of hominoidae, including humans, and causes an inflammation called hepatitis. It ranges in severity from a mild illness, lasting a few weeks (acute), to a serious long-term (chronic) illness that can lead to liver disease or liver cancer. The vaccine against the disease contains one of the viral envelope proteins, hepatitis B surface antigen (HBsAg). The FDA approved Hep B containing vaccines are Recombivax HB^{®} and Comvax^{®} by Merck, Engerix-B^{®} and Pediarix^{®} by GlaxoSmithKline Biologicals.

### Other antigens

The other antigens that the human race is concerned include *Haemophilus influenzae* (a, c, d, e, f serotypes and the unencapsulated strains), Hepatitis (A, C, D, E, F and G strains), meningitis A, B or C, Influenza, Pneumococci, Streptococci, anthrax, dengue, malaria, measles, mumps, rubella, BCG, Japanese encephalitis, Rotavirus, smallpox, yellow fever, typhoid, Singles, Varicella virus, and others.

### Combination vaccines

In spite of the long decades of research in the field of vaccines, the infectious diseases remain a threat to the human kind. Combination vaccines that protect against various diseases are very desirable since it reduces the number of shots given reduces the administration and production costs and improves the patient compliance as well. Such combination vaccines are generally better accepted.

However, the well documented phenomenon of the antigenic competition has complicated and hindered the development of the multivalent vaccines. This phenomenon refers to the observation that administering multiple antigens together often results in a diminished response to certain antigens relative to the immune response to these antigens when administered separately.

The earlier research has been focused on the development of the vaccines with multiple valencies directed towards different diseases and infections. One such well know vaccine combination comprises diphtheria (D), tetanus (T) and whole cell pertussis (wP) antigens to give a DTwP vaccine.

It is desirable to add other antigens to such a combination vaccine that would give protection against diseases caused by Hepatitis virus (Hep), *Haemophilus influenzae* (Hib) and polioviruses (IPV). It is also desirable to have antigens providing protection against other diseases added to the above said combination vaccines.

Quintanrix produced by GlaxoSmithKline Biologicals is a combination vaccine comprising DTwP, Hib and Hep B antigens, comprising thiomersal as a preservative. This vaccine was withdrawn before being marketed. Zilbrix ^{™} produced by GlaxoSmithKline Biologicals contains DTwP and Hep B. Zilbrix Hib ^{™} produced by GlaxoSmithKline Biologicals contains DTwP and Hep B and Hib, wherein the Hib is marketed in a separate vial, not containing the other antigenic components.

PCT Application WO2002005846 by Green Cross Vaccine provides a quadrivalent combination vaccine including diphtheria toxoid, tetanus toxoid, whole cell pertussis and hepatitis b surface antigen. The said invention relate to a vaccine preparation that combines Hep B with DTwP but is silent on the inclusion of other antigens such as Hib or IPV.

PCT Application WO2008028957 by GlaxoSmithKline Biologicals relates to a process of making a vaccine comprising low dose of IPV. However, it does not teach the preparation of a fully liquid vaccine comprising D, T, wP, IPV, Hib and Hep antigens, all together in a single vial, in fully liquid form, wherein the Hib is not substantially adsorbed on to any adjuvant.

United States Patent No. 6013264 by SmithKline Beecham Biologicals relates to a multivalent vaccine comprising HBsAg. The said invention relates to a combination vaccine in which the HBsAg is adsorbed on to aluminium phosphate. However, there is no specific disclosure of a combination vaccine comprising DTwP-IPV along with other antigens as Hib and Hep, in a fully liquid form.

PCT Application WO1998000167A1 by Connaught Lab provides a multivalent immunogenic composition for conferring protection in a host against disease caused by infection by *Bordetella pertussis, Clostridium tetani, Corynebacterium diphtheriae,* poliovirus and/or *Haemophilus influenzae.* The Hib b component of the vaccine, according to the specification is a lyophilized component that has to be reconstituted before mixing with the other components of the vaccine. Thus, there is no teaching in the specification related to a fully liquid combination vaccine comprising all the said antigens.

Lyophilization, also called as freeze drying, is a cost-intensive process that also causes a lot of stress to the proteins. When any component of the vaccine is lyophilized, at the time of administration of the vaccine, it is required to mix the lyophilizate with another liquid or the liquid component of the combination vaccine. This represents a supplementary constraint for the practitioner and presents a risk of it being carried out badly. It was then proposed to have a multi compartment syringe that would have the lyophilized component in one compartment and the liquid component of the vaccine in the other. However, such a syringe whose contents could be mixed at the time of administration of the vaccine, does not perform satisfactorily at the level of reducing the production costs as well as at the level of the operations to be carried out by the practitioner.

It is hence desirable to avoid this step of freeze drying and provide a combination vaccine that has all the components present together and in a fully liquid form. This would facilitate the administration of the vaccine, make it patient compliant and also reduce the production costs. It is thus desirable to have the Hib antigen added to the liquid component of the vaccine and thus have a fully liquid multivalent vaccine.

PCT Application W02004110480 by Glaxo SmithKline Biologicals relates to a vaccine comprising Hib b polysaccharide. The application states that simple mixing of the components of a combination vaccine is complicated by the fact that not all antigens can be effectively mixed together. It states that there is interference between the aluminium hydroxide of the DTP vaccine and PRP. The invention in WO'480 aims at minimizing this interference in such an extemporaneously-prepared combination vaccine wherein the PRP is pre-adsorbed onto aluminum phosphate. The invention further provides immunogenic compositions, vaccines and combination vaccines comprising PRP which is protected to some degree from immune interference. The inventors have found that the above can be achieved by incorporating a polyanionic polymer excipient with the vaccine comprising PRP.

However, use of the polyanionic polymer in the vaccine formulation may not be desirable as it may increase the cost of formulating the vaccine. Also, since a vaccine is finally intended for human use, it should ideally have the least components possible. The use of additional ingredients means addition of substances to the formulation to which the body may react and produce antibodies. Such a response of the body coming in contact with such components of the immunological preparation may not be desirable.

United States Patent No. 6333036 by Pasteur Merieux Serums, relates to vaccine compositions comprising capsular polysaccharide of *Haemophilus influenzae* type b or high molecular weight polyribosylribitol phosphate (PRP) coupled to tetanus anatoxin, as well as an aluminium-based adjuvant. The aluminium based adjuvants used in the invention have a point of zero charge of less than approximately 7.2. The patent however, does not particularly teach the preparation of a combination vaccine comprising DTwP-IPV along with other antigens as Hib and Hep, in a fully liquid form.

Though, the research is ongoing for making multivalent vaccine comprising various antigens that would afford protection against a number of diseases, they have not addressed the need for providing a stable combination vaccine comprising DTwP and IPV antigens along with other antigens such as Hib and Hep, in a fully liquid formulation. There are contrasting reports available regarding the antibody responses against particular antigens in children immunized by separate and co-administration of the combination vaccines and the Hib vaccine. There may be various reasons for such results including the vaccines being non-identical in their antigenic content, method of toxoiding, adjuvantation or the preservative used.

The currently known and available combination vaccines may not contain appropriate formulations of appropriate antigens in appropriate immunogenic forms for achieving desired levels of efficacy and immunogenicity in the susceptible human population, for a number of diseases in one shot. There is a need for a multi component vaccine that provides protection against various infections and is in liquid form so as to afford ease of administration and comfort of cost-effectiveness. It would be desirable to provide for a stable and efficacious multivalent vaccine against diseases caused by the infection caused by *Corynebacterium diphtheria, Clostridium tetani, Bordetella pertussis,* polioviruses, Hepatitis virus and *Haemophilus influenzae.* For such a vaccine to be effective, the criterion of seroprotection for each of the antigens of the vaccine needs to be fulfilled. For this there is a need to overcome the hurdles and the challenges posed by antigenic competition and interference. The present invention overcomes the limitations of prior arts and solves the related problems by providing a fully liquid combination vaccine formulation protecting against a plurality of diseases.

### SUMMARY OF THE INVENTION

The present invention relates to a fully liquid stable combination vaccine comprising Diptheria (D), Tetanus (T), Whole cell pertussis (wP), and IPV and optionally one or more antigens selected from the group of *Haemophilus influenzae* (Hib) and Hepatitis (Hep), such that administration of the vaccine can simultaneously immunize a subject against more than one pathogen. The invention in particular relates to a fully liquid stable combination vaccine comprising the antigens as indicated above and the methods for manufacturing the same.

The present invention is further directed towards a combination vaccine that comprises a plurality of the vaccine components that are suitable for the prevention, amelioration and treatment of multiple disease states that meet the criterion for the seroprotection for each of the said vaccine components.

### DETAILED DESCRIPTION OF THE INVENTION

The present invention is directed towards a fully liquid stable combination vaccine that comprises a plurality of the vaccine components that are suitable for the prevention, amelioration and treatment of multiple disease states that meet the criterion for the seroprotection for each of the said vaccine components.

The advantages of the present invention include a multivalent vaccine which can confer protection against a wide range of diseases and infections in a safe and efficacious manner. The vaccine of the invention provides immunogenicity to various diseases and infections without any interference of any of the antigen that is present in the vaccine. Thus, a single shot would confer immunogenicity against various diseases and infections, making the vaccine more patients compliant. Since a single shot would afford immunity against a number of infections and diseases, the cost of vaccination would be reduced. The vaccine of the present invention would be beneficial in the sense that it will reduce the number of visits to the vaccination centre and also the number of shots to be given for a number of different diseases and infections. This aspect of the invention would make it more useful and advantageous especially with the younger population who need to be vaccinated to confer immunity to a large number of infections and diseases. Thus, the present invention provides a vaccine that is more acceptable.

### Definitions

The term 'fully liquid' as used herein to describe the vaccine of the invention, refers to the state of the vaccine wherein all the components of the vaccine are in liquid state and there is no component of the vaccine that is provided in lyophilized or any other form so that it has to be mixed with the other components of the vaccine before administering it to a subject.

The term 'carrier protein' as used herein to describe the proteinic component to which the capsular polysaccharide (Hib) used in the vaccine is conjugated so as to convert the T-cell independent polysaccharide to T-cell dependent antigen.

The term 'adjuvant' as used herein to describe the non-antigenic component of the vaccine that enhances the immune response of the antigens of the vaccine by facilitating the contact between the antigen and the immune system by influencing the type and the quality of the immune response generated against an antigen. The adjuvant causes prolonged immune responses against the antigens and also may serve to decrease toxicity of certain antigens or provide solubility to certain antigens.

The term 'stable' used herein to describe the vaccine of the invention means that each of the antigens of the vaccine composition has a potency/ immunogenicity more than that set as the normal acceptance limit, after the incubation of the vaccine at 5±3°C for at least 1, preferably 12 and most preferably 24 months.

The term 'substantially' used herein to describe the amount of adsorption or coupling of the Hib on to any adjuvant, in the expression 'Hib is not substantially adsorbed on to any adjuvant', means that adsorption of Hib on to any adjuvant is less than 15%, and preferably less than 10%. The Hib antigen is not subjected to any step so as to intentionally adsorb it on to any adjuvant; the amount of the adsorption may be happening may be due to the contact between the antigen and the adjuvant and is not deliberate.

The term 'of about' used herein to describe the amount of each of the components present in the vaccine of the invention, means an amount of the said vaccine component that is present in amounts of preferably ±20%, more preferably ±10% and most preferably ±5% of the stated amount for that particular component.

The term 'about' as used herein to describe the time of stirring of the components of the mixture during the process of preparation of the vaccine of the invention, is preferably ±20%, more preferably ±10% and most preferably ±5% of the stated value.

The term 'immunologically active' used herein in reference to the combination vaccine of the invention means that the vaccine when administered to the subject is able to elicit antibodies against each of the antigen of the said combination so as to protect the vaccinee against the respective diseases or infections.

The term 'coupling or adsorbing' used herein with references to the antigens of the combination vaccine of the invention refers to any form of physical bonding between the antigen and the adjuvant.

### The vaccine of the invention

The present invention provides a fully liquid stable combination vaccine comprising Diptheria (D), Tetanus (T), Whole cell pertussis (wP), and Poliovirus antigens (IPV) and optionally one or more antigens selected from the group of *Haemophilus influenzae* (Hib) and Hepatitis (Hep).

One aspect of the invention provides a combination vaccine composition in which all the components of the vaccine are present together in liquid form in a single vial.

One further aspect of the invention relates to a fully liquid stable pentavalent vaccine comprising DTwP, Hib and IPV antigens.

Another aspect of the invention provides a fully liquid stable hexavalent vaccine comprising DTwP, Hib, Hep and IPV antigens.

The invention further provides that the diphtheria (D) and Tetanus (T) antigens are adsorbed on to aluminum phosphate.

According to one other aspect of the invention, the IPV strains are one or more Salk strains which may be selected from the group of Mahoney type 1, MEF type 2 and Saukett type 3 or one or more Sabin strains selected from the group of Sabin types 1 and 2.

According to a further aspect of the invention, the Hib antigen is conjugated to a carrier protein selected from a group comprising of tetanus toxoid (TT), diphtheria toxoid (DT), CRM 197 and outer membrane protein of *Neisseria meningitides* or any equivalents thereof, or any other known carriers.

According to another aspect of the invention, the Hib antigen is not substantially adsorbed on to any adjuvant.

According to one another aspect of the invention, the Hib antigen in the vaccine of the invention is derived from the capsular polysaccharide of Hib b strain.

One aspect of the invention provides that the Hep antigen is adsorbed on to aluminum phosphate.

One further aspect of the invention relates to the Hep antigen being derived from the Hepatitis B surface antigen (HBsAg); i.e. the surface antigen of the Hep B strain.

Another aspect of the invention provides that the vaccine of the invention comprises 2-phenoxyethanol as a preservative in the formulation.

According to one another aspect of the invention, the vaccine comprises D, T, wP, Hib b and Hep B antigens to give a pentavalent vaccine, and 2-phenoxyethanol is used as a preservative in the formulation, with the proviso that the vaccine does not comprise IPV antigens.

Further, other aspect of the invention relate to the process of manufacturing the combination vaccines of the invention.

One other aspect of the invention relates to the composition of the combination vaccines of the invention such that each of the antigens is present in an amount in the vaccine so as to elicit protective immune response against the said antigen.

### Antigens of the vaccine of the invention

Diphtheria is caused by *Corynebacterium diphtheriae,* a Gram-positive non-sporing aerobic bacterium. This organism expresses a prophage-encoded ADP-ribosylating exotoxin ('diphtheria toxin'), which can be treated (e.g. using formaldehyde) to give a toxoid. This toxoid is no longer toxic but still remains antigenic and is able to stimulate the production of specific anti-toxin antibodies after injection. The Diphtheria antigen preparation used in the vaccine of the invention preferably comprises Diphtheria toxoid.

Tetanus is caused by *Clostridium tetani,* a Gram-positive, spore-forming bacillus. This organism expresses an endopeptidase ('tetanus toxin'), which can be treated to give a toxoid that is no longer toxic. However it still remains antigenic and is able to stimulate the production of specific anti-toxin antibodies after injection. The Tetanus antigen preparation used in the vaccine of the invention preferably comprises Tetanus toxoid.

Pertussis or whooping cough is caused by *Bordetella pertussis.* The whole cell pertussis (wP) antigen of the invention may be prepared from the *Bordetella pertussis* strains that are likely to cause infections. The wP antigen of the invention may be inactivated by a number of ways such as use of chemical. However, inactivated *B. pertussis* (wP) used in the vaccine composition of the invention, preferably, is heat inactivated at 56 ±1°C, 30 minutes. The wP antigen preparation used in the vaccine of the invention is preferably made from *Bordetella pertussis* 134, 509 and 10536. The single Harvests of strains 10536, 509 and 134 are preferably mixed in the ratio of 1:1:1 ratio based upon their opacity.

*Haemophilus influenzae* is a Gram-negative coccobacillus which causes invasive bloodborne infections and meningitis. According to one embodiment of the invention, the Hib antigen, which is derived from the capsular polysaccharide, may be conjugated or coupled to carrier proteins. The carrier proteins used for the conjugation of the Hib antigen may be selected from comprising of tetanus toxoid (TT), diphtheria toxoid

(DT), CRM 197 and outer membrane protein of *Neisseria meningitides* or any equivalents thereof. Other suitable carrier proteins include, but are not limited to, synthetic peptides, heat shock proteins, pertussis proteins, cytokines, lymphokines, hormones, growth factors, artificial proteins comprising multiple human CD4+ T cell epitopes from various pathogen-derived antigens such as N19, protein D from *H.influenzae,* pneumococcal surface protein PspA, pneumolysin, iron-uptake proteins, toxin A or B from *C.difficile* and *S.agalactiae* proteins. The Hib antigen preparation used in the vaccine of the invention comprises Hib antigen preferably conjugated or coupled to tetanus toxoid.

The polysaccharide conjugate may be prepared by any known coupling technique. For example the polysaccharide can be coupled via a thioether linkage. This conjugation method relies on activation of the polysaccharide with 1-cyano-4-dimethylamino pyridinium tetrafluoroborate (CDAP) to form a cyanate ester. The activated polysaccharide may thus be coupled directly or via a spacer group to an amino group on the carrier protein. The conjugates can also be prepared by direct reductive amination methods. Another method involves the coupling of a cyanogen bromide (CNBr) activated polysaccharide derivatised with adipic acid hydrazide (ADH) to the protein carrier by carbodiimide condensation. Any other known method may be used to prepare the polysaccharide conjugate used in the vaccine of the invention.

According to an embodiment of the invention, the Hib antigen is not substantially adsorbed on to any adjuvant; preferably, degree of adsorption of the Hib antigen to an adjuvant is not more than 15%; more preferably, the degree of adsorption of the Hib antigen to an adjuvant is not more than 10%.

According to another embodiment of the invention relates to the Hib antigen not being subjected to deliberate or intentional adsorption on any adjuvant.

According to another preferred embodiment of the invention, the Hib antigen is derived from the capsular polysaccharide of the Hib b strain.

Hepatitis is caused by various Hepatitis strains such as A, B, C, D, E, F or G. Hepatitis B virus (HBV) is one of the major agents which cause viral hepatitis. The HBV virion consists of an inner core surrounded by an outer protein coat or capsid. The major component of the capsid is a protein known as HBV surface antigen or, more commonly, 'HBsAg'. When this antigen is administered to a vaccinee it stimulates the production of anti-HBsAg antibodies which protect against HBV infection. According to one preferred aspect of the invention, the Hepatitis (Hep) antigen preparation used in the vaccine of the invention comprises Hep antigens derived from the surface antigen of Hepatitis B strain (HBsAg).

For vaccine manufacture, HBsAg can be made either by purifying the antigen in particulate form from the plasma of chronic hepatitis B carriers, as large quantities of HBsAg are synthesized in the liver and released into the blood stream during an HBV infection or by expressing the protein by recombinant DNA methods. HBsAg for use in the vaccine of the invention may be prepared in either way.

Poliomyelitis is caused by the polio viruses. The vaccine of the invention may comprise Sabin (Sabin 1 and/ or Sabin 2) or Salk strains of Poliovirus. According to one preferred embodiment of the invention, the vaccine of the invention comprises Salk strains. There are 3 types of Salk strains that can cause poliomyelitis. The three types are similar and cause identical symptoms, but they are antigenically very different and infection by one type does not protect against infection by others. Salk Poliovirus includes 3 strains- Type 1 (e.g. Mahoney strain), poliovirus Type 2 (e.g. MEF-I strain), and poliovirus Type 3 (e.g. Saukett strain). According to a preferred embodiment of the invention, the vaccine of the invention may comprise one or more of the said Salk strains.

Polioviruses may be grown in cell culture. Vero cell line, which is a continuous cell line derived from monkey kidney, may be used to grow the polioviruses. After growth, virions may be purified using already known techniques. The inactivation of the viruses may be done. Quantities of poliovirus are typically expressed in the 'DU' unit (the "D-antigen unit"). The IPV antigen preparation used in the manufacturing of the vaccine of the invention is preferably prepared so as to comprise one or more strains that are used in the manufacturing of the vaccine. This bulk preparation is then used to formulate the vaccine of the invention.

### Non-antigenic components of the vaccine

Along with the antigenic components the vaccine may comprise a number of non-antigenic components that are pharmaceutically acceptable excipients. These include but are not restricted to pH modifiers, buffers, adjuvants, preservative, carrier and tonicity modifying agents.

### Adjuvants

The antigens of the final formulation may or may not be adsorbed on to an adjuvant. An adjuvant functions to stimulate the production of immunity against the vaccine ingredients, making the vaccine more effective.

Adjuvants may serve to:
- Bring the antigen in contact with the immune system and influence the type of immunity produced, as well as the quality of the immune response (magnitude or duration);
- Decrease the toxicity of certain antigens; and
- Provide solubility to some vaccines components.

Studies have shown that many aluminum-containing vaccines cause higher and more prolonged antibody responses than comparable vaccines without the adjuvant The benefit of adjuvants has usually been observed during the initial immunization series rather than with booster doses.

Aluminum based adjuvants are the most commonly used adjuvants. These adjuvants have also been approved by FDA for use in vaccines. There are three general types of aluminum-containing adjuvants:
- Aluminum hydroxide
- Alimunum phosphate
- Potassium aluminum sulfate (often called "Alum")

One embodiment of the invention relates to certain antigens of the vaccine being adsorbed on to aluminum phosphate and certain antigens of the vaccine being adsorbed on to aluminum hydroxide. Certain antigens of the invention may not be adsorbed or substantially adsorbed on to any adjuvant. The preference of the adjuvant for the adsorption of the said antigens gives the vaccine of the invention its characteristics. The preference of adsorption is described in more details in the section on 'Process for manufacturing of the vaccine of the invention', below.

### Preservatives

The vaccines are prone to contamination by bacteria. Thus, to avoid the potentially life threatening contamination with harmful microbes, that may be introduced in a vaccine incorporated during the event of accidental contamination, a preservative may be included in the composition of the vaccine while formulating it. The preservatives that have been used include Benzethonium chloride (Phemerol), thiomersal, Phenol and 2-phenoxyethanol (2-POE).

Thimerosal is a mercury-containing organic compound (an organomercurial) that has been used in many vaccines as a preservative. There are reports pertaining to certain allergic reactions to thiomersal primarily in the form of delayed-type local hypersensitivity reactions, including redness and swelling at the injection site. There are also conflicting reports on linking autism to mercury.

2-phenoxyethanol (2-POE) is also known as '1-hydroxy-2-phenoxyethane', '2-hydroxyethyl phenyl ether', 'ethyleneglycol phenyl ether', etc. The safety profile of 2-phenoxyethanol is better than that of mercurial preservatives (e.g. thiomersal). Thus, there is a need of avoiding thiomersal and using 2- phenoxyethanol in the vaccines.

Thus, another preferred embodiment of the invention relates to the use of 2-phenoxyethanol as a preservative in the vaccine composition. According to another preferred embodiment of the invention, the concentration of 2-phenoxyethanol is 5mg/ml of the vaccine.

### Tonicity modifying agents

To control to the tonicity of the vaccine composition, it is preferred to include a tonicity modifying agent in the formulation of the vaccine. These agents include but are not limited to salts (examples- NaCl, MgCl2, KCl, CaCl2), sugars (examples- dextrose, mannitol, lactose), amino acids (examples- Arginine, Glycine, Histidine) and Polyols (examples- Sucrose, Glycerol, Sorbitol). In a more preferred embodiment, a physiological salt such as sodium salt is used in the formulation of the vaccine. Sodium chloride (NaCl) is most preferably included in the vaccine composition of the invention.

### pH Modifiers and/or Buffers

Various pH modifiers known to person skilled in the art may be used to adjust the pH of the vaccine composition as desired, such as sodium hydroxide or hydrochloric acid. Various buffers such as sodium phosphate, potassium phosphate and citrate buffers may be used in the formulation of the vaccine.

### Composition of the vaccine of the invention

The composition of the vaccine of the invention is such that the vaccine of the invention is rendered immunogenic by the virtue of the amounts of each of the antigens contained in the vaccine. Each of the antigens in the vaccine of the invention is preferably in amounts so that the combination vaccine when administered to a subject, elicits immune response in the subject, against the said antigen of the composition. The combination vaccine of the invention comprises diphtheria (D), tetanus (T), whole cell pertussis (wP) and polio (IPV) antigens and optionally, other antigens selected from the group of *Haemophilus influenzae* (Hib) and Hepatitis (Hep).

According to one embodiment of the invention, the vaccine comprises D, T, wP, Hib b and IPV (Mahoney type 1, MEF Type 2 and the Saukett type 3), wherein D is present in an amount of about 1-40 Lf, T is present in an amount of about 1-25 Lf, wP is present in amount of about 1 - 30 IOU per 0.5 ml and Hib b is present in an amount of about 1 - 20 ug per 0.5 ml and the Mahoney type 1, MEF Type 2 and the Saukett type 3 strains are present in an amount of about 1-50 DU, 1 - 15 DU and 1-50 DU, respectively per 0.5 ml to form a pentavalent vaccine which would be stable, immunogenic and in form of a fully liquid formulation.

According to one preferred embodiment of the invention, the pentavalent vaccine of the invention comprises D, T, wP, Hib b and IPV wherein, D is present in an amount of about 20 Lf, T is present in an amount of about 7.5 Lf, wP is present in an amount of about 16 IOU per 0.5 ml, and Hib b is present in an amount of about 10 ug per 0.5 ml and the Mahoney type 1, MEF Type 2 and the Saukett type 3 strains are present in an amount of about 40 DU, 8 DU and 32 DU, respectively per 0.5 ml. Such a vaccine is formulated to give a fully liquid combination vaccine, would be stable and immunogenic when administered to a subject.

According to another embodiment of the invention, the hexavalent vaccine comprises D, T, wP, Hib b, Hep B and IPV (Mahoney type 1, MEF Type 2 and the Saukett type 3), wherein D is present in an amount of about 1-40 Lf, T is present in an amount of about 1-25 Lf, wP is present in amount of about 1-30 IOU per 0.5 ml, Hib b is present in an amount of about 1 - 20 ug and Hep B is present in an amount of about 1 - 20 ug per 0.5 ml and the Mahoney type 1, MEF Type 2 and the Saukett type 3 strains are present in an amount of about 1-50 DU, 1 - 15 DU and 1-50 DU, respectively per 0.5 ml to give a fully liquid stable combination vaccine, which would be immunogenic when administered to a subject.

According to another preferred embodiment of the invention, the hexavalent vaccine comprises D , T, wP, Hib b, Hep B and wherein D is present in an amount of about 20 Lf, T is present in an amount of about 7.5 Lf, wP is present in an amount of about 16 IOU per 0.5 ml, Hib b is present in an amount of about 10 ug and the Hep B is present in an amount of about 10 ug per 0.5 ml and the Mahoney type 1, MEF Type 2 and the Saukett type 3 strains are present in an amount of about 40 DU, 8 DU and 32 DU, respectively per 0.5 ml. Such a vaccine would be a stable vaccine capable of eliciting protective immune response against all the antigens of the composition, when administered to a subject.

One more aspect of the invention relates to a pentavalent vaccine comprising DTwP, Hib b and Hep B, wherein D is present in an amount of about 1-40 Lf, T is present in an amount of about 1-25 Lf, wP is present in amount of about 1-30 IOU, Hib b is present in an amount of about 2 - 20 ug and the Hep B is present in an amount of about 1 - 20 ug per 0.5 ml to give a fully liquid stable and immunogenic combination vaccine. According to one preferred aspect of the invention, the pentavalent vaccine comprises DTwP, Hib b and Hep B, wherein D is present in an amount of about 20 Lf, T is present in an amount of about 7.5 Lf, wP is present in an amount of about 16 IOU, Hib b is present in an amount of about 10 ug and Hep B is present in an amount of about 10 ug per 0.5 ml. Such a vaccine is stable and in form of fully liquid formulation. Such a vaccine would be a stable vaccine capable of eliciting protective immune response against all the antigens of the composition, when administered to a subject.

The invention further relates to a method of inducing immunological response to any of the antigen selected from the group of D, T, P, Hib, Hep or IPV comprising administering immunologically active amount of the vaccine of the invention.

According to another aspect of the invention, the aluminum content (Al⁺³) in the vaccine of the invention may not be more than preferably, 2 mg per 0.5 ml, more preferably 1 mg per 0.5 ml and most preferably 0.6 mg per 0.5 ml.

According to one another aspect of the invention the preferred amount of 2-phenoxyethanol in the combination vaccine of the invention may be 5 mg/ml.

### Process for manufacturing of the vaccine of the invention

One of the aspects of the invention relates to the process for manufacturing of the vaccine of the invention. The immunogenicity, the stability and the maintenance of the right form of the antigens in the immunogenic composition may depend on the way the composition has been formulated. This may include the sequence of addition of the antigens, the use of the specific adjuvants for certain antigens, the use of various parameters including agitation, temperature and pH.

One of the embodiments of the invention relates to the process of manufacturing the fully liquid combination vaccine comprising Diptheria (D), Tetanus (T), Whole cell pertussis (wP), and IPV and optionally one or more antigens selected from the group of *Haemophilus influenzae* (Hib) and Hepatitis (Hep), which comprises the steps of:
a) preparing a component I comprising a mixture of i) Diphtheria (D), ii) Tetanus (T) and iii) Whole cell Pertussis (wP) antigens,
b) optionally, preparing a component II comprising Hep adsorbed on to aluminium salts,
c) optionally, mixing the component II to component I to obtain a mixture
d) adding the above mixture [as obtained in a) or c)] to Hib, followed by addition of IPV antigens

According to one preferred embodiments of the invention, the D and T antigens are adsorbed on to aluminum phosphate.

One another preferred embodiments of the invention relates to the preparation of the component I which comprises the following steps:
a) transferring Aluminium phosphate gel into a vessel,
b) transferring Tetanus antigen preparation into the above vessel,
c) transferring Diphtheria antigen preparation under stirring into the said vessel
d) transferring 2-phenoxyethanol in the said vessel
e) transferring sodium chloride solution to the above said vessel under constant stirring
f) checking the pH and adjusting it in the range of 6.0- 6.5.
g) transferring wP antigen preparation under stirring, into the same vessel
h) checking the pH and adjusting it in the range of 6.5- 7.5.

The process further comprises the preparation of the component II comprising the following steps:
a) transferring Aluminium phosphate gel into a vessel.
b) transferring Hepatitis antigen preparation under stirring into the above vessel
c) transferring sodium chloride solution under stirring into the same vessel
d) transferring 2-phenoxyethanol preparation to the above said vessel
e) checking the pH and adjusting it to fall in the range of 6.0- 7.0

The process further involves the mixing of the component I and the component II comprises the step of transferring the contents of component II to the component I to obtain a mixture.

The process further involves the mixing of the above mixture to Hib and IPV antigens comprising the following steps:
a) transferring the above mixture to the Hib antigen preparation under stirring to obtain another mixture
b) transferring the above mixture to the IPV antigen followed by saline solution
c) checking the pH and adjusting it in the range of 6.5- 7.5

Another preferred embodiment of the invention relates to the addition of component I to the Hib and the IPV antigens, comprising the following steps:
a) transferring the Component I to Hib antigen preparation under stirring to obtain a mixture
b) transferring the above mixture to the IPV antigen preparation followed by saline solution
c) checking the pH and adjusting it in the range of 6.5- 7.5

Another preferred aspect of the invention relates to the preparation of the vaccine comprising the following steps:
a) preparing a component I comprising a mixture of i) Diphtheria (D), ii) Tetanus (T) and iii) Whole cell Pertussis (wP) antigens,
b) preparing a component II comprising Hep adsorbed on to aluminium salts,
c) mixing the component II to component I to obtain a mixture
d) adding the above mixture to Hib antigen

The process further comprises the preparation of the component I that comprises the following steps:
a) transferring Aluminium phosphate gel into a vessel,
b) transferring Tetanus antigen preparation under stirring into the above vessel
c) transferring Diphtheria antigen preparation into the said vessel
d) transferring 2-phenoxyethanol in the said vessel
e) transferring sodium chloride solution to the above said vessel under stirring
f) checking the pH and adjusting it in the range of 6.0- 6.5.
g) transferring wP antigen preparation into the same vessel,
h) checking the pH and adjusting it in the range of 6.5- 7.5

The process further relates to preparation of the component II comprising the following steps:
a) transferring Aluminium phosphate gel into a vessel.
b) transferring Hepatitis antigen preparation into the above vessel
c) transferring sodium chloride solution into the same vessel
d) transferring 2-phenoxyethanol preparation under stirring to the above said vessel
e) checking the pH and adjusting it to fall in the range of 6.0- 7.0

The process further relates to the mixing of the component I and the component II comprises the step of transferring the contents of component II to the component I to obtain a mixture.

The process further comprises the following steps:
a) transferring the above mixture to the Hib antigen preparation
b) checking the pH and adjusting it in the range of 6.5- 7.5

According to one preferred embodiment of the invention, the stirring as mentioned in any of the steps above is done at 150 rpm at 25 ± 2°C for about 30 minutes to 2 hours.

The following examples are used to further illustrate the present invention and advantages thereof. The following specific examples are given with the understanding that these are intended to be illustration without serving as a limitation on the scope of present invention.

### EXAMPLE I

### This example gives the composition and the process of manufacturing of a hexavalent vaccine as per one of the aspect of the invention

### A] Composition of the hexavalent vaccine as per the invention is as under:

Each 0.5 ml vaccine comprises the following:

**Table 1:**

| **COMPONENTS** | **AMOUNT** |
|---|---|
| Diphtheria Toxoid (DT) | 20 Lf |
| Tetanus Toxoid (TT) | 7.5 Lf |
| Inactivated *B. pertussis* antigen (wP) | 16 IOU |
| *Haemophilus influenzae* (Hib) b (capsular polysaccharide) antigen | 10 µg |
| Hepatitis (Hep) B Surface Antigen (HBsAg) | 10 µg |
| Inactivated Polio Virus (IPV) | |
| Type 1 | 40 D units |
| Type 2 | 8 D units |
| Type 3 | 32 D units |
| **Other Ingredients:-** | |
| Aluminum Content (as Aluminum Phosphate) | *NMT 0.6 mg of Al⁺³ |
| 2-Phenoxy Ethanol | 2.5 mg |

| | |
|---|---|
| *NMT- not more than. | |

### B] The process of manufacturing of the hexavalent vaccine as per the invention is as under:

### Formulation procedure for Component I

The aluminium phosphate gel was transferred into a vessel, followed by the Tetanus antigen and the Diphtheria antigen under stirring to obtain a mixture. 2-phenoxyethanol was then mixed with the above mixture in the said vessel Sodium chloride solution was then added to the above. The pH of the mixture was checked and adjusted to fall in the range of 6.0- 6.5. The wP antigen was then transferred into the same vessel. The pH of the mixture was then checked and adjusted to fall in the range of the range of 6.5- 7.5.

### Formulation procedure for Component II

The aluminium phosphate gel was transferred into a vessel, followed by Hepatitis B antigen. The sodium chloride solution then transferred into the same vessel, followed by 2-phenoxyethanol. The pH of the mixture was checked and adjusted to fall in the range of 6.0- 7.0.

### Mixing of Component I and Component II

This step was carried out by transferring the contents of the component II to the component I to obtain a mixture

### Addition of Hib b and IPV bulk

Further, the above mixture of the component I and II was mixed with the Hib b antigen preparation to obtain a mixture. This was further added to the IPV antigen to obtain a mixture and saline solution were added to the above mixture to obtain a hexavalent vaccine, the pH was checked and adjusted to fall in the range of 6.5- 7.5.

### EXAMPLE II

### This example gives the composition and the process of manufacturing a pentavalent vaccine as per one of the aspect of the invention

### A] Composition of the pentavalent vaccine as per the invention is as under

Each 0.5 ml vaccine comprises the following:

**Table 2:**

| **COMPONENTS** | **AMOUNT** |
|---|---|
| Diphtheria Toxoid (DT) | 20 Lf |
| Tetanus Toxoid (TT) | 7.5 Lf |
| Inactivated *B. pertussis* antigen (wP) | 16 IOU |
| *Haemophilus influenzae* (Hib) b (capsular polysaccharide) antigen | 10 µg |
| Inactivated Polio Virus (IPV) | |
| Type 1 | 40 D units |
| Type 2 | 8 D units |
| Type 3 | 32 D units |
| **Other Ingredients:-** | |
| Aluminum Content (as Aluminum Phosphate) | *NMT 0.6 mg of Al⁺³ |
| 2-Phenoxy Ethanol | 2.5 mg |

| | |
|---|---|
| *NMT- not more than | |

### B] The process of manufacturing of the pentavalent vaccine as per the invention is as under:

### Formulation procedure for Component I

The aluminium phosphate gel was transferred into a vessel, followed by the Tetanus antigen and the Diphtheria antigen under stirring to obtain a mixture. 2-phenoxyethanol was then mixed with the above mixture in the said vessel Sodium chloride solution was then added to the above. The pH of the mixture was checked and adjusted to fall in the range of 6.0- 6.5. The wP antigen was then transferred into the same vessel. The pH of the mixture was then checked and adjusted to fall in the range of the range of 6.5- 7.5.

### Addition of Hib b and IPV bulk

The component I was added to the Hib b antigen to obtain a mixture, which was then mixed with the IPV antigen preparation and saline solution to obtain a pentavalent vaccine. The pH of the mixture was adjusted to fall in the range of 6.5- 7.5.

### Example III

### This example gives the composition and the process of manufacturing a pentavalent vaccine as per one of the aspect of the invention

### A] Composition of the pentavalent vaccine as per the invention is as under

Each 0.5 ml vaccine comprises the following:

**Table 3:**

| **COMPONENTS** | **AMOUNT** |
|---|---|
| Diphtheria Toxoid (D) | 20 Lf |
| Tetanus Toxoid (T) | 7.5 Lf |
| Inactivated *B. pertussis* antigen (wP) | 16 IOU |
| *Haemophilus influenzae* (Hib) b (capsular polysaccharide) antigen | 10 µg |
| Hepatitis (Hep) B Surface Antigen (HBsAg) | 10 µg |

| **Other Ingredients:-** | |
|---|---|
| Aluminum Content (as Aluminum Phosphate) | *NMT 0.6 mg of Al⁺³ |
| 2-Phenoxy Ethanol | 2.5 mg |

| | |
|---|---|
| *NMT- not more than. | |

### B] The process of manufacturing of the pentavalent vaccine as per the invention is as under:

### Formulation procedure for Component I

The aluminium phosphate gel was transferred into a vessel, followed by the Tetanus antigen and the Diphtheria antigen under stirring to obtain a mixture. 2-phenoxyethanol was then mixed with the above mixture in the said vessel Sodium chloride solution was then added to the above. The pH of the mixture was checked and adjusted to fall in the range of 6.0- 6.5. The wP antigen was then transferred into the same vessel. The pH of the mixture was then checked and adjusted to fall in the range of the range of 6.5- 7.5.

### Formulation procedure for Component II

The aluminum phosphate gel was transferred into a vessel, followed by Hep B antigen preparation. The sodium chloride solution was then transferred into the same vessel, followed by 2-phenoxyethanol preparation to obtain a mixture. The pH was adjusted to fall in the range of 6.0- 7.0.

### Mixing of Component I and Component II

This step involved the transfer of the contents of component II to the component I to obtain a mixture.

### Addition of Hib b bulk

The above mixture of the components I and II were then mixed with Hib b antigen preparation to obtain a pentavalent vaccine. The pH of the mixture obtained was checked and adjusted to fall in the range of 6.5- 7.5.

### EXAMPLE IV

### This example gives a brief on the in-vivo Potency testing carried out for Diphtheria, Tetanus, Whole Cell Pertussis, Haemophilus influenzae type (Hib) b, Hepatitis B and Inactivated Polio antigens and the Stability data or the Potency for the same.

### A] In-vivo Potency testing carried out for Diphtheria, Tetanus, Whole Cell Pertussis, Haemophilus influenzae type (Hib) b, Hepatitis B and Inactivated Polio antigens

### 1. Diphtheria Toxoid

| | |
|---|---|
| Animal species required | : Guinea pigs |
| No. of animals required for 1 batch | : 116 (48 for test, 48 for reference & 20 for LD₅₀) |
| Route of vaccine administration | : Subcutaneous |
| Volume of injection | : 1.0 ml |
| No. of days animals are housed | : 28 |

Potency of Diphtheria toxoid was determined in Guinea pigs by lethal challenge method. In this method, three dilutions each of the test and the reference vaccine were prepared such that the middle dilution contains the ED₅₀ dose that saves at least or more than 50% of the test animals. Sixteen Guinea pigs for every dilution of test vaccine & reference vaccine were inoculated and after 28 days the test animals were subcutaneously challenged with Diphtheria toxin containing 100 LD₅₀. A group of twenty Guinea pigs were kept unimmunized for the titration of the Diphtheria Toxin & this group of guinea pigs were inoculated with different dilutions of Diphtheria toxin, 5 guinea pigs for every dilution. Test was completed in 33 days. Further calculations were done by using PROBIT. The sample passes the diphtheria potency test if it contains ≥ 30 IU/Single Human Dose.
- The test vaccine should fulfill linearity & parallelism with reference vaccine.
- Fiducial limit of estimated potency should lie between 50 to 200%.
- Estimated potency should not be less than 30 I.U. per single human dose.
- The limit of 95% confidence interval of estimate of potency should be within 50-200% unless the lower limit of the 95% confidence interval of the estimated potency should be greater than 30 I.U. per dose

### 2. Tetanus Toxoid

| | |
|---|---|
| Animal species required | : Swiss albino mice |
| No. of animals required for 1 batch | : 116 (48 for test, 48 for reference & 50 for LD₅₀) |
| Route of vaccine administration | : Subcutaneous |
| Volume of injection | : 0.5 ml |
| No. of days animals are housed | : 28 |

Potency of Tetanus toxoid was determined in Swiss mice by lethal challenge method. In the test, three dilutions each of the test and the reference vaccine were prepared such that the middle dilution contains the ED₅₀ dose that saves at least or more than 50% of the test animals. A group of sixteen Swiss mice for every dilution of test vaccine as well as reference vaccine was inoculated and after 28 days of housing the test animals were challenged with Tetanus toxin containing 100 LD₅₀. A group of twenty Swiss mice were kept unimmunized for the titration of the Tetanus Toxin & were inoculated with 5 mice for 4 dilutions of LD₅₀ titration. Test was completed in 33 days. Further calculations were carried by using PROBIT. Potency sample passes the tetanus potency test if it contains ≥ 60 IU/Single Human Dose.
- The test vaccine should fulfill linearity & parallelism with reference vaccine.
- Fiducial limit of estimated potency should lie between 50 to 200%.
- Estimated potency should not be less than 60 I.U. per single human dose.

The limit of 95% confidence interval of estimate of potency should be within 50-200% unless the lower limit of the 95% confidence interval of the estimated potency is greater than 60 I.U. per dose.

### 3. Whole Cell Pertussis antigen

| | |
|---|---|
| Animal species required | : Swiss mice |
| No. of animals required for 1 batch | : 194 (72 for test, 72 for reference & 50 for LD₅₀) |
| Route of vaccine administration | : Intraperitoneal |
| Volume of injection | : 0.5 ml |
| No. of days animals are housed | : 14 |

Potency test of Pertussis antigen was carried out in Swiss mice by Mouse protection test. In this method, three dilutions each of the test and the reference vaccine were prepared in normal saline such that the middle dilution contains the ED₅₀ dose that saves at least or more than 50% of the test animals. A group of 24 Swiss mice were immunized per dilution and then all the immunized animals are intracerebrally challenged with 0.03 ml of challenge dose (approximately 100,000 organisms per 0.03 ml) on 14^{th} day. After the challenge the test animals were observed for 14 days and death and survival of the test animals were noted. Death occurring in first three days after challenge was taken as non-specific. Further calculations were done by using PROBIT. The sample passes the pertussis potency test if it contains ≥ 4 IU/Single Human Dose (SHD) and the lower fiducial limit is not less than 2 IU/SHD.

### 4. Hemophilus influenza (Hib) type b antigen

| | |
|---|---|
| Animal species required | : Swiss mice |
| No. of animals required for 1 batch | : 16 (8 for immunization, 8 serving as control) |
| Route of vaccine administration | : Subcutaneous |
| Volume of injection | : 0.5 ml |
| No. of days animals are housed | : 35 |

Potency test of Hib b antigen was carried out in Swiss mice by ELISA method. In this test two groups (test and control group) each of 8 Swiss mice were selected. Each mouse of test group was subcutaneously injected with 0.5 ml of 1:4 diluted vaccines and control group was left uninoculated. Booster dose was given on 10^{th} and 20^{th} day and final blood collection is done on 35^{th} day. Serum samples were tested for antibody against Hib by ELISA method. The sample passes the Hib potency test if ≥ 50% mice in test group are seroconverted.

### 5. Hepatitis B Surface Antigen

| | |
|---|---|
| Animal species required | : Balb C mice |
| No. of animals required for 1 batch | : 110(50 for sample, 50 for reference, 10 for Placebo) |
| Route of vaccine administration | : Intraperitoneal |
| Volume of injection | : 1.0 ml |
| No. of days animals are housed | : 28 |

Potency test of Hepatitis B Surface Antigen was carried out in Balb/C mice. In this test, five two-fold dilutions each of reference and test vaccine were prepared and ten mice were intraperitoneally inoculated per dilution. Ten mice were inoculated with diluent and served as placebo. The inoculated mice were bled on 28^{th} day of inoculation and sera was separated taking care that no hemolysis occurs. The sera samples were tested for antibody titer against Hepatitis B by ELISA. The sample passes the Hepatitis B potency test if the upper limit of its relative potency ≥ 1.

### 6. Inactivated Polio Vaccine (IPV)

| | |
|---|---|
| Animal species required | : Wistar rats |
| No. of animals required for 1 batch | : 100 (50 for test vaccine, 50 for reference Vaccine) |
| Route of vaccine administration | : Intramuscular |
| Volume of injection | : 0.5 ml |
| No. of days animals are housed | : 21 |

Potency test of IPV was carried out in RIVM TOX rats. Five three fold dilutions each of reference and test vaccine were prepared and ten rats were intramuscularly injected with 0.5 ml of each dilution. Test animals were bled 21 days after the immunization and serum samples were collected carefully avoiding lysis of RBCs. Each serum sample was tested for antibody titer against type 1, type 2 and type 3 serotypes of Polio virus by serum neutralization test.

The test is not valid unless:
- the median effective dose (ED50) for both the test and reference vaccines lies between the smallest and the largest doses given to the animals;
- the statistical analysis shows no significant deviation from linearity or parallelism;
- the fiducial limits of the estimated relative potency fall between 25% and 400% of the estimated potency.

### B] Stability data or the potency of the antigens in the vaccine of the invention

1) The tests were carried out as given in Example IV A] for all the antigens of the hexavalent vaccine of the Example I. The results are as tabulated in the table below.

**Table 4:**

| **Components** | | **Acceptance Limits** | **Long Term Study** (5°C ± 3°C) | |
|---|---|---|---|---|
| | | | **6 months** | **12 months** |
| **Diphtheria toxoid** (**DT**) (Potency) | | ≥ 60 IU/ml | 157.28 | - |
| **Tetanus Toxoid** (**TT**) (Potency) | | ≥ 120 IU/ml | 209.57 | - |
| **Inactivated B. pertussis (wP)** | | ≥ 8 IU/ml | 9.44 | 9.69 |
| (Potency) | | | | |
| **Capsular polysaccharide (Hib b) antigen** (Potency) | | ≥ 50 % | 62.5 | 75 |
| **Hepatitis (Hep) B surface antigen** (**HBsAg**) (Potency) | | ≥ 1.0 | 1.56 | 1.50 |
| **Inactivate d Polio Virus** (**IPV**) (Potency) | Type 1 | Potency of the test Vaccine should not be less than the Ref. Vaccine | - | Complies |
| | Type 2 | | - | Complies |
| | Type 3 | | - | Complies |

Thus, it can be seen that even after the long term storage of the vaccine at 5 ± 3°C, the vaccine antigens are still potent / have immunogenicity above the acceptance limits.

2) The tests were carried out as given in Example IV A] for all the antigens of the pentavalent vaccine of the Example II. The results are as tabulated in the table below.

**Table 5:**

| **Components** | | **Acceptance Limits** | **Long Term Study** (5°C ± 3°C) | |
|---|---|---|---|---|
| | | | **6 months** | **12 months** |
| **Diphtheria toxoid** (**DT**) (Potency) | | ≥ 60 IU/ml | 166.22 | -- |
| **Tetanus Toxoid** (**TT**) (Potency) | | ≥ 120 IU/ml | 208.77 | -- |
| **Inactivated *B*. *pertussis*** (**wP**) (Potency) | | ≥ 8 IU/ml | 9.37 | 10.5 |
| **Capsular polysaccharide** (**Hib b) antigen** (Potency) | | ≥ 50% | 87.5 | 75 |
| **Inactivated Polio Virus** (**IPV**) (Potency) | Type 1 | Potency of the test Vaccine should not be less than the Ref. Vaccine | - | Complies |
| | Type 2 | | - | Complies |
| | Type 3 | | - | Complies |

Thus, it can be seen that even after the long term storage of the vaccine at 5 ± 3° C, the vaccine antigens are still potent / have immunogenicity above the acceptance limits.

## Claims

1. A fully liquid stable combination vaccine, comprising Diptheria (D), Tetanus (T), whole cell pertussis (wP), Hib, and Hep B antigens, and 2-phenoxyethanol as a preservative, with the proviso that the vaccine does not comprise IPV antigens, wherein Hib is conjugated to a carrier protein selected from a group comprising of tetanus toxoid, diphtheria toxoid, CRM 197 and outer membrane protein of *Neisseria meningitides* or any equivalents thereof, or any other known carriers and wherein Hib is not substantially adsorbed on to any adjuvant.

2. The vaccine as claimed in the claim 1, wherein the Diptheria (D), Tetanus (T) antigens are adsorbed on to aluminum phosphate.

3. The vaccine as claimed in the claim 1, wherein the Hib antigen is derived from the capsular polysaccharide of Hib b strain.

4. The vaccine as claimed in claim 1, wherein the Hep antigen is adsorbed on to aluminum phosphate.

5. The vaccine as claimed in the claim 1, wherein the Hep antigen is derived from the surface antigen of the Hep B strain.

6. The vaccine as claimed in claim 1, wherein Diptheria (D) is present in an amount of about 1-40 Lf, Tetanus (T) is present in an amount of about 1-25 Lf, whole cell pertussis (wP) is present in amount of about 1-30 IOU, the Hib b antigen is present in an amount of about 2-20 µg, and the Hep B antigen is present in an amount of about 1 - 20 µg per 0.5 ml.

7. The vaccine as claimed in claim 6, wherein Diptheria (D) is present in an amount of about 20 Lf, Tetanus (T) is present in an amount of about 7.5 Lf, wP is present in an amount of about 16 IOU, the Hib b antigen is present in an amount of about 10 µg, and Hep B is present in an amount of about 10 µg per 0.5 ml.

8. A process for manufacturing a fully liquid stable combination vaccine, as claimed in the claim 1, comprising the steps of:
a) preparing a component I comprising a mixture of i) Diphtheria (D), ii) Tetanus (T) and iii) whole cell Pertussis (wP) antigens,
b) preparing a component II comprising Hep B adsorbed on to aluminium salts,
c) mixing the component II to component I to obtain a mixture, and
d) adding the above mixture to Hib antigen.

9. The process as claimed in the claim 8, wherein Diptheria (D) and Tetanus (T) antigens are adsorbed on to aluminum phosphate.

10. The process as claimed in claim 8, wherein the preparation of the component I comprises the following steps:
a) transferring of Aluminium phosphate gel into a vessel,
b) transferring Tetanus antigen preparation under stirring into the above vessel,
c) transferring Diphtheria antigen preparation into the said vessel,
d) transferring 2-phenoxyethanol,
e) transferring sodium chloride solution to the above said vessel,
f) checking the pH and adjusting it in the range of 6.0- 6.5,
g) transferring wP antigen preparation into the same vessel, and
h) checking the pH and adjusting it in the range of 6.5- 7.5.

11. The process as claimed in the claim 8, wherein the preparation of the component II comprises the following steps:
a) transferring aluminium phosphate gel into a vessel,
b) transferring Hep B antigen preparation into the above vessel,
c) transferring sodium chloride solution into the same vessel,
d) transferring 2-phenoxyethanol preparation to the above said vessel, and
e) checking the pH and adjusting it to fall in the range of 6.0- 7.0.

12. The process as claimed in the claim 8, wherein the mixing of the component I and the component II comprises the step of transferring the contents of component II to the component I to obtain a mixture.

13. The process as claimed in the claim 12, further comprising the following steps:
a) transferring the mixture obtained to the Hib antigen preparation, and
b) checking the pH and adjusting it in the range of 6.5- 7.5.

14. The combination vaccine as claimed in any one of claims 1 to 7, for use in a method of inducing immunological response to any of the antigen selected from the group of Diptheria (D), Tetanus (T), whole cell pertussis (wP), Hib and Hep antigens in a subject.
